# FASCICULE DE BREVET EUROPEEN

(11) **EP 2 242 500 B1**
(45) Date de publication et mention de la délivrance du brevet: **31.10.2018**
(21) Numéro de dépôt: 09706796.1
(22) Date de dépôt: 28.01.2009
(51) Int. Cl.: A61K 36/8905

(54) **UTILISATION D'UN ACTIF ISSU DE CYPERUS ESCULENTUS POUR SON ACTION CUTANEE ANTI-AGE**
VERWENDUNG EINES WIRKSTOFFES AUS CYPERUS ESCULENTUS ZUM SCHUTZ VOR HAUTALTERUNG
USE OF AN ACTIVE AGENT DERIVED FROM CYPERUS ESCULENTUS FOR THE CUTANEOUS ANTI-AGEING ACTION THEREOF

(30) Priorité: 28.01.2008 FR 0850502
(43) Date de publication de la demande: 27.10.2010
(73) Titulaire: Société Industrielle Limousine d'Application Biologique Dite SILAB, 19130 Objat (FR)
(72) Inventeur: PAUFIQUE, Jean, 19130 Objat (FR)
(74) Mandataire: Aquinov
(86) Numéro de dépôt international: PCT/FR2009/050128
(87) Numéro de publication internationale: WO 2009/095615

(56) Documents cités:
- WO-A-92/20322
- PARKER MARY L ET AL: "Esterified phenolics of the cell walls of Chufa (Cyperus esculentus L.) tubers and their role in texture" décembre 2000 (2000-12), JOURNAL OF AGRICULTURAL AND FOOD CHEMISTRY, VOL. 48, NR. 12, PAGE(S) 6284-6291 , XP009105009 ISSN: 0021-8561 le document en entier
- DATABASE WPI Week 200213 Thomson Scientific, London, GB; AN 2002-096213 & RU 2 176 516 C1 (BUZLAMA V S) 10 December 2001 (2001-12-10)

## Description

La présente invention concerne l'utilisation cosmétique d'un principe actif issu de Cyperus esculentus pour lutter contre le vieillissement cutané, préférentiellement en agissant sur les marqueurs spécifiques du derme.

L'invention se rapporte également à un principe actif issu de Cyperus esculentus, à son procédé d'obtention, ainsi qu'aux compositions anti-âges comprenant au moins un principe actif issu de Cyperus esculentus.

Le derme est considéré et reconnu comme la cible principale des actifs cosmétiques pour lutter contre le vieillissement cutané.

Le derme comprend une diversité de molécules matricielles organisées en une structure très dynamique qui détermine les propriétés biomécaniques du tissu et qui joue un rôle primordial au cours des évènements liés à la vie cellulaire. Cet alliage macromoléculaire compose deux compartiments dermiques séparés par un plexus vasculaire : le derme papillaire ou papilles dermiques et le derme réticulaire.

Le derme papillaire est une zone critique du tissu cutané de par son rôle clé dans la cohésion et dans les propriétés biomécaniques de la peau, mais également parce qu'il est la première cible du vieillissement dermique intrinsèque.

L'objectif de la présente invention est de proposer des produits cosmétiques capables de lutter efficacement contre le vieillissement cutané, pour aider à rajeunir et à revitaliser la peau en considérant les aspects du vieillissement de la peau sur les différentes couches cutanées, préférentiellement en agissant sur les marqueurs spécifiques du derme papillaire.

Pour y répondre, la présente invention vise l'utilisation d'un principe actif issu de Cyperus esculentus pour la préparation d'une composition cosmétique et/ou dermopharmaceutique destinée à lutter contre le vieillissement cutané.

Le derme papillaire, localisé sous les crêtes épidermiques, tient son nom de sa surface en papilles qui forment des saillies.

Il est constitué d'un tissu conjonctif lâche renfermant des fibrilles fines hétérotypiques composées de collagène I et de collagène de type III et V, qui, contrairement à celles du derme réticulaire, sont peu organisées. Ces collagènes matriciels sont impliqués dans l'architecture matricielle, la résistance, la cohésion et la flexibilité de la peau.

Le derme papillaire contient également des fibres oxytalan ou microfibrilles qui sont constituées majoritairement de fibrillines, grosses glycoprotéines. Ces microfibrilles forment un réseau perpendiculaire ancré dans la membrane basale de la jonction dermo-épidermique (JDE). Le réseau microfibrillaire du derme papillaire assure les propriétés élastiques de la peau et crée également une zone tampon dotée d'un rôle « amortisseur » puissant de la JDE en cas de fortes contraintes mécaniques.

Une étude conduite par le déposant a montré que la synthèse de la fibrilline-1 par les fibroblastes papillaires sénescents est significativement réduite par rapport aux fibroblastes papillaires normaux témoins.

C'est pourquoi, un objectif de la présente invention est notamment d'agir sur la synthèse de fibrilline-1, de façon à restaurer l'organisation fonctionnelle du réseau microfibrillaire d'oxytalan du derme papillaire.

Contrairement au derme réticulaire, le derme papillaire contient aussi des collagènes non fibrillaires qui lui sont spécifiques, les collagènes de type XII et XVI, qui contribuent à l'échafaudage matriciel de cette région.

Le collagène XVI présente une structure complexe et est localisé uniquement au niveau de la zone superficielle du derme papillaire. Il est exclusivement associé aux fibres oxytalan en interagissant avec la fibrilline-1. Il assure ainsi l'ancrage des fibres oxytalan à la membrane basale de la JDE.

Or, une étude conduite par le déposant a montré que l'expression du collagène XVI est significativement réduite sur les fibroblastes papillaires sénescents comparés aux fibroblastes papillaires normaux.

Un autre objectif de la présente invention est donc d'agir sur la synthèse du collagène XVI spécifique du derme papillaire, et d'agir ainsi sur l'extensibilité et l'organisation fonctionnelle du réseau microfibrillaire d'oxytalan du derme papillaire.

L'expression du collagène XII est restreinte au derme papillaire. En liant les fibrilles de collagène I aux autres composés matriciels, le collagène XII régule les propriétés biomécaniques du tissu cutané : déformabilité et contraction des fibres de collagène en favorisant les glissements de fibres les unes par rapport aux autres.

Or, une étude conduite par le déposant a montré que l'expression du collagène XII est significativement réduite sur les fibroblastes papillaires sénescents comparés aux fibroblastes papillaires normaux.

Aussi, la présente invention se propose aussi d'agir sur la synthèse du collagène XII, spécifique du derme papillaire, dans l'optique d'agir sur la déformabilité du derme papillaire.

Par ailleurs, on sait que l'expression du collagène I est significativement réduite sur les fibroblastes papillaires sénescents en comparaison aux fibroblastes papillaires normaux.

Aussi, la présente invention propose d'agir sur la synthèse du collagène I, dans l'optique d'avoir un effet sur l'architecture matricielle et la résistance du derme papillaire.

En outre, l'arrangement tridimensionnel des fibres de collagène est facilité par la présence de molécules matricielles telles que les protéoglycannes mais également le collagène VI. La particularité du collagène VI est sa multiplicité d'actions. Il se lie en effet avec un grand nombre de cellules via des récepteurs type intégrines ainsi qu'avec de multiples molécules matricielles (collagène IV, fibronectine, biglycan, MAGP-1).

L'expression du collagène VI est significativement réduite sur les fibroblastes papillaires sénescents.

C'est pourquoi la présente invention se propose d'agir sur la synthèse du collagène VI, dans l'optique d'améliorer la cohésion et la flexibilité du derme papillaire. Pour répondre à son objectif, la présente invention vise donc l'utilisation d'un principe actif issu de Cyperus esculentus.

Cyperus esculentus est une herbe vivace de la famille des Cyperacées, qui mesure de 30 à 40 cm de hauteur et qui possède des rhizomes grêles épaissis en tubercules ovoïdes ou subglobuleux de la grosseur d'une noisette, des feuilles linéaires, plus courtes que la tige de 5 mm de large, vert pâle, glabres avec 3 à 5 bractées, et des épillets verts ou jaunâtres, longs de 8 à 15mm avec des glumes de 3 à 4 stries de chaque côté. C'est une plante largement répandue dans le monde, notamment en Afrique, sur le pourtour méditerranéen et en Amérique du Nord.

Le Cyperus esculentus ou souchet est principalement connu pour ses propriétés culinaires. Ses tubercules au goût d'amande peuvent être consommés crus ou secs et le lait extrait de ces tubercules sert à la préparation de spécialités culinaires en particulier en Espagne. En outre, il était autrefois utilisé par les égyptiens et les américains natifs comme aliment de base substitut de céréales et dans certains pays d'Afrique comme élément nutritif important.

On sait aussi que l'huile extraite des tubercules Cyperus esculentus peut servir d'ingrédient pour la fabrication de savons et de lubrifiants, ou encore en tant que parfum comme au Burkina Faso.

Cyperus esculentus a également des vertus médicinales. Il présente des propriétés aphrodisiaque, carminative, digestive, diurétique, emménagogue, stimulant et tonique. En Ayurveda il est utilisé, comme une autres espèce, le Cyperus rotundus, pour le traitement des flatulences, des indigestions, des diarrhées, de la dysenterie et de la soif excessive. Au Maroc notamment, le souchet comestible passait autrefois pour un puissant spermatogène, aphrodisiaque et galactogène.

L'utilisation de l'espèce Cyperus rotendus est également connue pour une application cosmétique dépigmentante de la peau ou des cheveux, comme décrit dans la demande de brevet WO-92/20322, mais aucune référence au traitement de vieillissement cutané n'y est faite ni à l'utilisation spécifique de l'espèce Cyperus esculentus.

La présente invention vise l'utilisation d'un principe actif issu de Cyperus esculentus dans ou pour la préparation d'une composition cosmétique et/ou dermopharmaceutique à administration par voie topique destinée à lutter contre le vieillissement cutané. Préférentiellement, ledit principe actif est capable d'agir sur le derme papillaire, en particulier :
- en limitant la dégradation des fibres d'oxytalan et en stimulant la synthèse de fibrilline-1, composant majoritaire des fibroblastes papillaires, de façon à restaurer l'organisation fonctionnelle du réseau microfibrillaire d'oxytalan du derme papillaire, et/ou
- en stimulant l'expression des collagènes fonctionnels XVI spécifiques du derme papillaire, et ainsi améliorer l'extensibilité de la peau, et/ou
- en stimulant l'expression des collagènes fonctionnels XII spécifiques du derme papillaire, et ainsi améliorer la déformabilité de la peau, et/ou
- en stimulant l'expression des collagènes matriciels I, de façon à améliorer l'architecture matricielle et la résistance de la peau, et/ou
- en stimulant l'expression des collagènes matriciels VI, et améliorer la cohésion et la flexibilité de la peau.

Avantageusement, selon l'invention l'utilisation d'au moins un principe actif issu de Cyperus esculentus permet de lutter contre l'apparition des rides, de diminuer la fatigabilité de la peau liée au vieillissement cutané, et/ou d'augmenter son élasticité et/ou sa déformabilité.

Le principe actif issu de Cyperus esculentus selon l'invention est destiné à être incorporé au sein de compositions cosmétiques et/ou dermopharmaceutiques.

La composition selon l'invention peut contenir de 0,01 à 20 % d'au moins un principe actif issu de Cyperus esculentus.

L'administration d'une composition cosmétique et/ou dermopharmaceutique contenant au moins un principe actif issu de Cyperus esculentus selon l'invention est effectuée par voie topique.

La composition selon l'invention peut se présenter sous forme de crèmes, émulsions huile-dans-eau, eau-dans-huile ou émulsions multiples, solutions, suspensions ou encore poudres. Préférentiellement, elle se présente sous forme de gel, de solution ou d'émulsion et comprend :
- entre 0,01% et 20% d'au moins un principe actif issu de Cyperus esculentus, comme agent anti-vieillissement,
- au moins un ingrédient additionnel, choisi parmi les matières premières additionnelles utilisées en cosmétique, de préférence la glycérine, et
- un véhicule dermatologiquement acceptable.

Selon un autre aspect, l'invention vise un principe actif particulier issu de Cyperus esculentus destiné à lutter contre le vieillissement cutané et capable d'agir sur la synthèse d'au moins une molécule fibreuse du derme papillaire. Ce principe actif est susceptible d'être obtenu par un procédé comprenant les étapes suivantes :
- solubilisation de poudre de tubercules de Cyperus esculentus dans l'eau,
- hydrolyses enzymatiques successives, au moyen de deux carbohydrases,
- séparation des phases soluble et insoluble, et
- inactivation enzymatique par traitement thermique.

L'invention couvre également un procédé d'obtention de ce principe actif.

La présente invention est maintenant décrite en détail en s'appuyant sur un exemple de principe actif issu de Cyperus esculentus et des résultats de tests.

### 1/ PROCEDE D'OBTENTION D'UN PRINCIPE ACTIF SELON L'INVENTION :

Le principe actif issu de Cyperus esculentus utile selon l'invention, est susceptible d'être obtenu par un procédé comprenant les étapes suivantes :
- solubilisation de poudre de tubercules de Cyperus esculentus dans l'eau,
- hydrolyses enzymatiques successives,
- séparation des phases soluble et insoluble, et
- inactivation enzymatique par traitement thermique.

Les enzymes utilisées sont des carbohydrases. Il peut s'agir d'enzymes purifiées, d'enzymes mono-constituant ou encore d'un mélange de différentes enzymes. A titre d'exemple on peut citer l'amidon dégradé par alpha-amylase, la beta-1,4 amylase, la glucosamylase ; les hémicellulases comme l'arabinase, l'arabinofuranosidase, la xynalase, la 1,3 ou 1,4-beta xylosidase, la beta-1,4-galactanase, l'apha-galactosidase, la beta-galactosidase, la mannase ; les celluloses comme la cellobiohydrolase, l'endoglucanase ; les pectinases comme la rhamnogalacturonase, la rhamnopyranohydrolase, la polygalacturonase, la glucuronisidase ; ou encore la beta-glucanase ou la beta-glucosidase. L'inactivation enzymatique peut être suivie d'une ou plusieurs étapes de filtration(s) et/ou de concentration(s). Préférentiellement, l'inactivation enzymatique est suivie par les étapes suivantes :
- filtration
- purification de la fraction active par ultrafiltration, et
- filtration et filtration stérilisante.

Le principe actif peut être obtenu sous forme liquide ou sous forme de poudre par atomisation ou lyophilisation.

### 2/ CARACTERISATION D'UN PRINCIPE ACTIF SELON L'INVENTION :

Selon un deuxième aspect, l'invention concerne un principe actif cosmétique anti-age, issu de Cyperus esculentus, susceptible d'être obtenu par la mise en oeuvre du procédé présenté précédemment.

De façon préférée le principe actif est obtenu à partir de poudre de Tubercules de Cyperus esculentus.

Préférentiellement, le principe actif selon l'invention est sous forme liquide.

Il peut être défini par les caractéristiques exposées ci-après.

### 2-1/ Matières sèches :

On mesure le taux de matières sèches par passage à l'étuve à 105°C en présence de sable d'un échantillon de poids initial donné jusqu'à obtention d'un poids constant.

Le taux de matières sèches d'un principe actif issu de Cyperus esculentus selon l'invention est compris entre 10 et 250 g/l, plus particulièrement entre 38 et 52 g/l.

### 2-2/ Mesure du pH :

Le pH mesuré par la méthode potentiométrique à température ambiante conduit à des valeurs comprises entre 3 et 7, plus particulièrement entre 4,5 et 5,5.

### 2-3/ Détermination de la teneur en sucres totaux

On utilise la méthode de DUBOIS. En présence d'acide sulfurique concentré et de phénol, les sucres réducteurs donnent un composé jaune orangé. A partir d'une gamme étalon, on peut déterminer le taux de sucres totaux d'un échantillon.

Le taux de sucres totaux d'un principe actif issu de Cyperus esculentus selon l'invention est compris entre 8 et 240 g/l, préférentiellement entre 32 et 50 g/l.

### 2-4/ Caractérisation des sucres monomères

La caractérisation des sucres monomères d'un principe actif issu de Cyperus esculentus selon l'invention est réalisée par chromatographie liquide haute performance (CLHP).

Les résultats obtenus montrent qu'un principe actif issu de Cyperus esculentus selon l'invention est composé, en ordre décroissant, de glucose, de galactose, d'arabinose, de xylose, de mannose et de rhamnose. En particulier sa fraction glucidique comprend au moins 95% de glucose.

### 2-5/ Caractérisation des carbohydrates

La détermination de la taille des carbohydrates d'un principe actif issu de Cyperus esculentus selon l'invention est réalisée par chromatographie liquide haute performance.

Le chromatogramme obtenu montre la présence de plus de 90% de monosaccharides de masse moléculaire inférieure à 180Da et de moins de 10% d'oligosaccharides de masse molaire comprise entre 300 et 2500Da. La fraction glucidique du principe actif issu de Cyperus esculentus selon l'invention est donc composée essentiellement de glucose présent sous forme de monosaccharides et d'oligosaccharides.

### 2-6/ Identification de la fraction active

La fraction active du principe actif issu de Cyperus esculentus, agissant sur l'expression des ARNm (Acide RiboNucléique messager) codant pour le collagène I est constituée majoritairement par des oligosaccharides de poids moléculaire supérieur à 900Da.

### 3. EVALUATION DE L'EFFET D'UN PRINCIPE ACTIF ISSU DE CYPERUS ESCULENTUS SELON L'INVENTION

L'extrait utilisé pour les études qui suivent est un extrait de Cyperus esculentus obtenu par la mise en oeuvre d'un procédé comprenant les étapes suivantes :
- solubilisation de poudre de tubercules de Cyperus esculentus dans l'eau à raison de 50g/l,
- hydrolyses enzymatiques successives au moyen de deux carbohydrases,
- séparation des phases soluble et insoluble par décantation pendant une nuit,
- inactivation enzymatique par traitement thermique à 90°C pendant 3 heures,
- purification de la fraction active par ultrafiltration, et
- filtration stérilisante sur un filtre de 0,22µm.

Le principe actif obtenu présente les caractéristiques suivantes :
- aspect : liquide limpide
- couleur : ambré clair
- matières sèches : 50,1g/l
- sucres totaux : 36,2g/l sous forme de monosaccharides et d'oligosaccharides,
- protéines totales : 6,0g/l, (le taux de protéines totales est obtenu par la méthode Kjedhal - Official method of analysis of the A.O.C. 1975, 12th ed W Horwitz, E.D., New-York, p15-60-)
- cendres : 7,8g/l (la teneur en cendres brutes est déterminée par la pesée des résidus issus de l'incinération à 550°C dans un four à moufle électrique)
- polyphénols = 0,1g/l (le dosage des composés phénoliques est obtenu à l'aide de ferricyanure de potassium en se rapportant à une gamme étalon d'acide gallique de 40 à 120mg/l).

Cet exemple d'extrait n'est bien entendu pas limitatif.

### 3.1 Evaluation de l'effet in vitro

Le protocole utilisé pour mettre en place les modèles cellulaires des études qui suivent, fait référence à la publication « JM SORELL et al., Journal of cellular physiology, 200 : 134-145 (2004) ».

### 3.1.1 - Effet d'un principe actif issu de Cyperus esculentus sur la synthèse du réseau microfibrillaire d'oxytalan

### a- Quantification sur fibroblastes papillaires normaux et sénescents par Western Blot

Cette étude a pour objectif d'évaluer l'effet d'un principe actif issu de Cyperus esculentus selon l'invention sur la synthèse du réseau microfibrillaire d'oxytalan en utilisant comme marqueur la fibrilline-1, le composant majeur de ces fibres.

L'étude est réalisée par Western Blot sur fibroblastes humains papillaires normaux et sénescents dont le vieillissement est induit par passages successifs.

Le protocole opératoire est le suivant.

A J1, les fibroblastes sont ensemencés dans du milieu MEM complet, puis incubés à 37°C.

A J2, les cellules sont traitées. Pour les fibroblastes papillaires normaux et pour les fibroblastes papillaires sénescents, le milieu de culture est éliminé et remplacé par du milieu MEM contenant ou non un principe actif issu de Cyperus esculentus selon l'invention à 0,5% et 1%.

Les cellules sont ensuite incubées à 37°C.

A J4, les surnageants cellulaires sont récupérés et concentrés, puis stockés à -80°C.

Un Western Blot est ensuite réalisé pour doser la fibrilline-1. Les résultats obtenus sont présentés dans le tableau suivant :

| | Synthèse de fibrilline-1 (%) | |
|---|---|---|
| | Fibroblastes papillaires normaux | Fibroblastes papillaires vieillis |
| Témoin non traité | 100 | 69 |
| Principe actif issu de Cyperus esculentus 0,5% | 116 | 105 |
| Principe actif issu de Cyperus esculentus 1% | 123 | 113 |

La synthèse de la fibrilline-1 par les fibroblastes papillaires sénescents est significativement réduite de 31% par rapport aux fibroblastes papillaires normaux témoins.

On constate que testé à 1%, un principe actif issu de Cyperus esculentus selon l'invention augmente la synthèse de le fibrilline-1 par les fibroblastes papillaires normaux de 23% et restaure significativement la synthèse de la fibrilline-1 par les fibroblastes papillaires sénescents à un taux similaire à celui des fibroblastes papillaires normaux témoins.

### b- Visualisation sur explants de peau humaine par coloration histologique

L'objectif de cette étude est d'évaluer l'effet d'un principe actif issu de Cyperus esculentus selon l'invention sur la synthèse des fibres d'oxytalan, réseau spécifique du derme papillaire.

L'étude est réalisée sur explants de peaux humaines obtenus d'un donneur jeune et d'un donneur âgé. La visualisation est obtenue après coloration histologique.

Le protocole opératoire consiste dans un premier temps à traiter les explants de peau puis à réaliser la coloration des fibres d'oxytalan.

A J1, des punchs de 8mm de diamètre sont réalisés à partir de plastie humaine et maintenus en survie dans du milieu de culture.

De J2 à J8, ces explants sont traités topiquement avec un placebo ou le principe actif selon l'invention formulé à 4% une fois par jour pendant 7 jours.

A J9, les explants sont récupérés et congelés. Des coupes sont ensuite réalisées.

On réalise ensuite la coloration des fibres d'oxytalan sur les coupes selon le protocole décrit par « Ph.D G GODEAU et al. Analytical and Quantitative Cytology and Histology, 8, 4 : 321-325 (1986) ».

On visualise ensuite la coloration à l'aide d'un microscope couplé à un système d'analyse d'images. Les fibres d'oxytalan apparaissent spécifiquement en bleu foncé et sont perpendiculaires à la membrane basale.

Les résultats histologiques étant qualitatifs, trois niveaux d'expression ont été définis :

| | |
|---|---|
| - faible détection de coloration | + |
| - Moyenne détection de coloration | ++ |
| - Forte détection de coloration | +++ |

Les résultats obtenus sont présentés dans le tableau suivant :

| | Synthèse des fibres d'oxytalan |
|---|---|
| Explant 28 ans traité avec le placebo | +++ |
| Explant 55 ans traité avec le placebo | + |
| Explant 55 ans traité avec le principe actif selon l'invention 4% | ++ |

Sur un explant de peau de donneur âgé, le nombre de fibres d'oxytalan est réduit et leur organisation perpendiculaire à la JDE est fortement altérée en comparaison à l'explant de donneur jeune.

Ces résultats montrent que testé ex-vivo à 4% sur un explant de donneur âgé, un principe actif issu de Cyperus esculentus selon l'invention permet de rebooster la synthèse du réseau microfibrillaire d'oxytalan et de restaurer son organisation fonctionnelle.

### 3.1.2 - Effet sur l'expression des collagènes

L'objectif de cette étude est d'évaluer l'effet d'un principe actif issu de Cyperus esculentus selon l'invention sur sa capacité à augmenter l'expression des ARNm codant pour les collagènes XVI, XII, VI et I.

Cette étude est réalisée par PCR quantitative sur fibroblastes humains papillaires normaux et sénescents, selon le protocole opératoire décrit en suivant.

A J1, les fibroblastes papillaires humains normaux ou sénescents sont ensemencés puis incubés à 37°C.

A J3, les cellules sont traitées avec un principe actif issu de Cyperus esculentus selon l'invention à 0,5% ou 1% ou avec une molécule de référence, du TGFβ-1 à 1ng/ml. Elles sont ensuite incubées à 37°C.

A J5, les cellules sont récupérées et les ARN totaux extraits. Les ARN sont reverse-transcripts et les ADN (Acides DesoxyriboNucléiques) complémentaires obtenus sont analysés par PCR quantitative.

Les résultats obtenus sont présentés dans les tableaux suivants :

| | Taux de collagène XVI (%) | |
|---|---|---|
| | Fibroblastes papillaires normaux | Fibroblastes papillaires sénescents |
| Témoin non traité | 100 | 31 |
| TGFβ-1 à 1ng/ml | 172 | 156 |
| Principe actif issu de Cyperus esculentus 0,5% | 101 | 114 |
| Principe actif issu de Cyperus esculentus 1% | 109 | 131 |

| | Taux de collagène XII (%) | |
|---|---|---|
| | Fibroblastes papillaires normaux | Fibroblastes papillaires sénescents |
| Témoin non traité | 100 | 10 |
| TGFβ-1 à 1ng/ml | 135 | 66 |
| Principe actif issu de Cyperus esculentus 0,5% | 96 | 35 |
| Principe actif issu de Cyperus esculentus 1% | 99 | 60 |

| | Taux de collagène VI (%) | |
|---|---|---|
| | Fibroblastes papillaires normaux | Fibroblastes papillaires sénescents |
| Témoin non traité | 100 | 22 |
| TGFβ-1 à 1ng/ml | 156 | 143 |
| Principe actif issu de Cyperus esculentus 0,5% | 95 | 130 |
| Principe actif issu de Cyperus esculentus 1% | 98 | 162 |

| | Taux de collagène I (%) | |
|---|---|---|
| | Fibroblastes papillaires normaux | Fibroblastes papillaires sénescents |
| Témoin non traité | 100 | 17 |
| TGFβ-1 à 1ng/ml | 212 | 213 |
| Principe actif issu de Cyperus esculentus 0,5% | 97 | 55 |
| Principe actif issu de Cyperus esculentus 1% | 98 | 77 |

On constate que l'expression des ARNm des collagènes XVI, XII, VI et I par les fibroblastes papillaires humains sénescents sont réduits de 69%, 90%, 78% et 83% respectivement, par rapport aux fibroblastes papillaires humains normaux.

Les résultats obtenus montrent qu'un principe actif issu de Cyperus esculentus selon l'invention dosé à 1% restaure l'expression des ARNm des collagènes XVI et VI et tend à restaurer celle des collagènes XII et I à un taux similaire à celui des fibroblastes papillaires normaux témoins.

### 3.2 Evaluation de l'effet in vivo

### 3.2.1 - Effet sur le ressort cutané

Cette étude a pour objectif d'évaluer in vivo l'efficacité d'un principe actif issu de Cyperus esculentus selon l'invention formulé à 4% en émulsion contre placebo sur le ressort de la peau.

L'étude est réalisée sur 20 volontaires sains de sexe féminin d'âge compris entre 45 et 73 ans.

Les mesures sont réalisées au niveau du bas du visage à l'aide d'un Cutomètre : la peau est aspirée par une sonde à dépression constante pendant une durée constante. La profondeur de pénétration de la peau dans la sonde est mesurée par deux prismes optiques, ce qui permet d'obtenir des courbes à partir desquelles il est possible de calculer divers paramètres caractéristiques des propriétés mécaniques de la peau. Parmi ces paramètres, la présente étude consiste à observer les paramètres suivants :
- R4 qui représente la fatigabilité de la peau (si R4 diminue, l'effet « fatigue de la peau » est moins important),
- R5 qui représente l'élasticité de la peau (si R5 augmente, l'élasticité de la peau augmente)
- R8 qui représente la capacité de la peau à revenir son état initial, (si R8 augmente, la capacité de la peau à revenir à son état initial augmente).

Le protocole de l'étude est exposé en suivant.

Entre J-15 et J0, les volontaires appliquent deux fois par jour une crème placebo.

A J0, on détermine deux zones cutanées symétriques au niveau du bas du visage et on mesure les propriétés biomécaniques de la peau sur ces deux zones.

Entre J0 et J27, les volontaires appliquent bi-quotidiennement le placebo et le produit contenant le principe actif issu de Cyperus esculentus selon l'invention.

A J28, on effectue des mesures des propriétés biomécaniques de la peau sur les deux zones du visage étudiées.

Entre J28 et J55, les volontaires appliquent bi-quotidiennement le placebo et le produit contenant le principe actif issu de Cyperus esculentus selon l'invention.

A J56, on effectue des mesures des propriétés biomécaniques de la peau sur les deux zones du visage étudiées.

Les résultats obtenus sont présentés dans le tableau ci-dessous :

| | Variation par rapport au placebo (%) | |
|---|---|---|
| | J28 | J56 |
| R4 | -11% | -13% |
| R5 | +5% | +15% |
| R8 | +7% | +10% |

Le principe actif issu de Cyperus esculentus selon l'invention permet d'augmenter l'élasticité de la peau de 15% et la capacité de la peau à revenir à son état initial de 10%.

De plus, on constate que des applications biquotidiennes du principe actif issu de Cyperus esculentus selon l'invention pendant 56 jours permettent de diminuer la fatigue de la peau de 13% par rapport au placebo.

### 3.2.2 - Etude des propriétés anti-rides

Le but de l'étude est d'évaluer in vivo l'efficacité anti-rides d'un principe actif issu de Cyperus esculentus selon l'invention formulé à 4% en émulsion contre placebo au niveau des pattes d'oie.

L'étude est réalisée sur 20 volontaires de sexe féminin d'âge compris entre 45 et 73 ans.

L'efficacité anti-rides est mesurée par l'intermédiaire d'empreintes siliconées réalisées au niveau des pattes d'oie des volontaires. L'analyse de ces empreintes à l'aide d'un profilomètre muni d'un analyseur d'images permet d'obtenir trois paramètres : le nombre de rides, la surface totale ridée et la longueur totale des rides.

L'étude est réalisée selon le protocole ci-dessous.

Entre J-15 et J0, les volontaires appliquent deux fois par jour une crème placebo.

A J0, on détermine deux zones cutanées symétriques au niveau des pattes d'oie, une destinée à être traitée par le placebo, l'autre par le principe actif formulé, et on prend les empreintes sur ces deux zones.

Entre J0 et J27, le principe actif et le placebo sont appliqués deux fois par jour.

A J28, les empreintes sont prises sur les deux zones étudiées. Entre J28 et J55, le principe actif formulé et le placebo sont appliqués deux fois par jour.

A J56, les empreintes sont prises sur les deux zones étudiées.

Les résultats obtenus pour le principe actif par rapport à ceux obtenus pour le placebo sont exprimés en pourcentage dans le tableau qui suit :

| | Variation/Placebo (%) | |
|---|---|---|
| | J28 | J56 |
| Nombre de rides | -21 | -25 |
| Surface totale ridée | -26 | -40 |
| Longueur totale | -19 | -30 |

On constate qu'après 28 jours d'applications biquotidiennes en comparaison au placebo, le principe actif issu de Cyperus esculentus selon l'invention formulé à 4% en émulsion diminue à la fois le nombre de rides de 21%, la surface totale ridée de 26% et la longueur totale des rides de 19%. Après 56 jours la diminution est de 25% pour le nombre de rides, de 40% pour la surface totale ridée et de 30% pour la longueur totale des rides.

### 4. EXEMPLES DE COMPOSITION

La présente invention couvre aussi les compositions cosmétiques et/ou dermopharmaceutiques incluant au moins un principe actif issu de Cyperus esculentus selon la présente invention dans différentes formes galéniques, adaptées à l'administration par voie topique cutanée.

Ces compositions peuvent se présenter notamment sous forme de crèmes, émulsions huile-dans-eau, émulsions eau-dans-huile, émulsions multiples, solutions, suspensions ou poudres. Elles peuvent être plus ou moins fluides et avoir l'aspect d'une crème, d'une lotion, d'un lait, d'un sérum, d'une pommade, d'un gel, d'une pâte ou d'une mousse, ou sous forme solide.

Ces compositions contiennent entre 0,01 et 20% en poids de principe(s) actif(s) issu(s) de Cyperus esculentus selon la présente invention.

On peut citer des formulations ayant montré une stabilité physique incluant 5% de principe actif selon l'invention.

### Gel limpide :

- Carbopol : 0,5% avec Triéthanolamine : qsp pH=6,5
- Glycérol : 10%
- Propylène glycol : 10%
- Conservateur : 1,0%
- Principe actif : 5,0%
- Eau : 73,5%

### Gel opaque :

- Sépigel 305 : 3%
- Lanol 99 : 12%
- Conservateur : 1,0%
- Principe actif : 5,0%
- Eau : 79%

### Gel émulsionné :

- Montanov 202 : 3,0%
- Isopropyl palmitate : 10%
- Conservateur : 1,0%
- Sépigel 305 : 2%
- Principe actif : 5,0%
- Eau : 79%

### Emulsion non ionique :

- Montane 60 : 2,0%
- Montanox 60 : 4,0%
- Isopropyl myristate : 8%
- Paraffin wax 130/135 : 3%
- Conservateur : 1,0%
- Principe actif : 5,0%
- Eau : 77%

### Emulsion anionique :

- Acide stéarique : 7,0% Triéthanolamine qsp pH=8
- Ritaphyl ICS : 20%
- Conservateur : 1,0%
- Principe actif : 5,0%
- Eau : 67%

### Emulsion cationique :

- Quaternium-82 : 5,0%
- Alcool cétylique : 1%
- Gemseal 60 : 8%
- Alcool cétéarylique : 1%
- PEG 100 stéarate : 1%
- Conservateur : 1,0%
- Principe actif : 5,0%
- Eau : 78%

De plus, des tests ont montré la compatibilité du principe actif avec les matières premières utilisées en cosmétique : épaississants, émulsionnants, solvants.

On peut également citer des exemples de compositions cosmétiques anti-âges incluant le principe actif selon l'invention. Les exemples de composition qui suivent sont obtenus par mélange des différents composants. Les quantités indiquées sont données en pourcentage de poids.

### 4.1 Exemple d'un sérum contour de l'oeil

La formulation est la suivante :

| | |
|---|---|
| - Simulgel EG (Seppic) : | 2% |
| - DUB 1632 (Stéarinerie Dubois) : | 1% |
| - DC 345 (Dow Corning) : | 2% |
| - Ritox 59 (Rita) : | 1% |
| - Glycerox 767 HC (Croda) : | 4% |
| - DUB MCT 5545 (Stéarinerie Dubois) | 2% |
| - Conservateur : | 1% |
| - Principe actif selon l'invention : | 4% |
| - Eau : | qsp 100% |

### 4.2 Exemple d'une crème visage siliconée

La formulation est la suivante :

| | |
|---|---|
| - Glycérine : | 3% |
| - Butylène Glycol : | 2% |
| - Propylène Glycol : | 2% |
| - Phytosqualane (Sophim) : | 8% |
| - DUB STG 30 AE (Stéarinerie Dubois) : | 4% |
| - DC 345 (Dow Corning) : | 25% |
| - Huile de Parme (Sictia) : | 4% |
| - DUB Heliocristal (Stéarinerie Dubois) : | 4% |
| - Conservateur : | 1% |
| - Principe actif selon l'invention : | 4% |
| - Eau : | qsp 100% |

### 4.3 Exemple d'une crème de nuit

La formulation est la suivante :

| | |
|---|---|
| - Emulgade 1000NI (Henkel) : | 1% |
| - Montane 60 (Seppic) : | 1% |
| - Rita CA (Rita) : | 1% |
| - Ritacane (Rita) : | 4% |
| - Sophim MC 30 (Sophim) : | 6% |
| - Sucrose ester SP30 C (Sisterna) : | 1% |
| - Conservateur : | 1% |
| - Principe actif selon l'invention : | 4% |
| - Eau : | qsp 100% |

### 4.4 Exemple d'un gel hydratant lactescent

La formulation est la suivante :

| | |
|---|---|
| - Ultrez 21 (Noveon) : | 0,2% |
| - Glycérine : | 9% |
| - EDT 2020 (Noveon) : | 0,27% |
| - Blanose 9M31F (Hercules) : | 0,2% |
| - DUB Synersol (Stéarinerie Dubois) : | 6% |
| - Montanox 80 (Seppic) : | 3% |
| - NaOH : | qsp pH=6,2 |
| - Conservateur : | 1% |
| - Principe actif selon l'invention : | 4% |
| - Eau : | qsp 100% |

## Revendications

1. Utilisation d'au moins un principe actif issu de poudre de tubercules de Cyperus esculentus dans ou pour la préparation d'une composition cosmétique et/ou dermopharmaceutique à administration par voie topique pour lutter contre le vieillissement cutané.

2. Utilisation d'au moins un principe actif issu de Cyperus esculentus selon la revendication 1, **caractérisée en ce que** ledit principe actif est capable d'agir sur la synthèse d'au moins une molécule fibreuse spécifique du derme papillaire choisie parmi les collagènes fonctionnels XII et/ou XVI, les fibres d'oxytalan et la fibrilline-1 du derme papillaire.

3. Utilisation d'au moins un principe actif issu de Cyperus esculentus selon l'une des revendications précédentes, **caractérisée en ce que** ledit principe actif est capable d'augmenter l'expression des collagènes fonctionnels XII et/ou XVI et/ou des fibres d'oxytalan et/ou de la fibrilline-1 du derme papillaire.

4. Utilisation d'au moins un principe actif issu de Cyperus esculentus selon l'une des revendications précédentes, **caractérisée en ce que** ledit principe actif est capable d'augmenter l'expression des collagènes matriciels I et/ou VI.

5. Principe actif, adapté à une utilisation selon l'une quelconque des précédentes revendications, susceptible d'être obtenu par un procédé comprenant les étapes suivantes :
- solubilisation de poudre de tubercules de Cyperus esculentus dans l'eau,
- hydrolyses enzymatiques successives, au moyen de deux carbohydrases,
- séparation des phases soluble et insoluble, et
- inactivation enzymatique par traitement thermique.

6. Principe actif selon la revendication 5, **caractérisé par** :
- un taux de matières sèches compris entre 10 et 250 g/l,
- un pH compris entre 3 et 7, et
- une teneur en sucres totaux comprise entre 8 et 240 g/l.

7. Principe actif selon la revendication 5 ou 6, **caractérisé par** un taux de matières sèches compris entre 38 et 52 g/l.

8. Principe actif selon l'une des revendications 5 à 7, **caractérisé par** un pH compris entre 4,5 et 5,5.

9. Principe actif selon l'une des revendications 5 à 8, **caractérisé par** une teneur en sucres totaux comprise entre 32 et 50 g/l.

10. Procédé d'obtention d'un principe actif selon l'une des revendications 5 à 9, comprenant les étapes suivantes :
- solubilisation de poudre de tubercules de Cyperus esculentus dans l'eau,
- hydrolyses enzymatiques successives, au moyen de deux carbohydrases,
- séparation des phases soluble et insoluble,
- inactivation enzymatique par traitement thermique, et
- filtration(s).

11. Composition cosmétique et/ou dermopharmaceutique à administration par voie topique destinée à lutter contre le vieillissement cutané, contenant entre 0,01% et 20% d'au moins un principe actif issu de poudre de tubercules de Cyperus esculentus.

12. Composition cosmétique et/ou dermopharmaceutique selon la revendication 11, **caractérisée en ce qu'**elle se présente sous forme de crème, émulsion huile-dans-eau, émulsion eau-dans-huile, émulsions multiples, solution, suspension ou poudre.

13. Composition cosmétique et/ou dermopharmaceutique selon la revendication 11 ou 12, **caractérisée en ce que** le principe actif est un principe actif selon l'une des revendications 5 à 9.

## Patentansprüche

1. Verwendung wenigstens eines aus dem Pulver der Knolle von Cyperus esculentus hervorgegangenen Wirkstoffs in der oder für die Herstellung einer kosmetischen und/oder dermopharmazeutischen Zusammensetzung zur topischen Anwendung zur Bekämpfung der Hautalterung.

2. Verwendung wenigstens eines aus Cyperus esculentus hervorgegangenen Wirkstoffs nach Anspruch 1, **dadurch gekennzeichnet, dass** der Wirkstoff in der Lage ist, auf die Synthese wenigstens eines für die papilläre Dermis spezifischen Fasermoleküls einzuwirken, das unter den funktionellen Kollagenen XII und/oder XVI, den Oxytalanfasern und Fibrillin-1 der papillären Dermis ausgewählt ist.

3. Verwendung wenigstens eines aus Cyperus esculentus hervorgegangenen Wirkstoffs nach einem der vorhergehenden Ansprüche, **dadurch gekennzeichnet, dass** der Wirkstoff in der Lage ist, die Expression der funktionalen Kollagene XII und/oder XVI und/oder der Oxytalanfasern und/oder von Fibrillin-1 der papillären Dermis zu steigern.

4. Verwendung wenigstens eines aus Cyperus esculentus hervorgegangenen Wirkstoffs nach einem der vorhergehenden Ansprüche, **dadurch gekennzeichnet, dass** der Wirkstoff in der Lage ist, die Expression der Matrixkollagene I und/oder VI zu steigern.

5. Wirkstoff, der zu einer Verwendung nach einem der vorhergehenden Ansprüche eingerichtet ist und dazu geeignet ist, durch ein Verfahren gewonnen zu werden, das die folgenden Verfahrensschritte umfasst:
- Lösen des Pulvers der Knollen von Cyperus esculentus in Wasser,
- aufeinanderfolgende enzymatische Hydrolysen mit Hilfe von wenigstens zwei Carbohydrasen,
- Trennung der löslichen und unlöslichen Phasen, und
- enzymatische Deaktivierung durch Wärmebehandlung.

6. Wirkstoff nach Anspruch 5, **gekennzeichnet durch**:
- einen Trockensubstanzgehalt zwischen 10 und 250 g/l,
- einen pH-Wert zwischen 3 und 7, und
- einen Gesamtzuckergehalt zwischen 8 und 240 g/l.

7. Wirkstoff nach Anspruch 5 oder 6, **gekennzeichnet durch** einen Trockensubstanzgehalt zwischen 38 und 52 g/l.

8. Wirkstoff nach einem der Ansprüche 5 bis 7, **gekennzeichnet durch** einen pH-Wert zwischen 4,5 und 5,5.

9. Wirkstoff nach einem der Ansprüche 5 bis 8, **gekennzeichnet durch** einen Gesamtzuckergehalt zwischen 32 und 50 g/l.

10. Verfahren zur Gewinnung eines Wirkstoffs nach einem der Ansprüche 5 bis 9, mit den folgenden Verfahrensschritten:
- Lösen des Pulvers der Knollen von Cyperus esculentus in Wasser,
- aufeinanderfolgende enzymatische Hydrolysen mit Hilfe von wenigstens zwei Carbohydrasen,
- Trennung der löslichen und unlöslichen Phasen,
- enzymatische Deaktivierung durch Wärmebehandlung, und
- Filterung(en).

11. Kosmetische und/oder dermopharmazeutische Zusammensetzung zur topischen Anwendung zur Bekämpfung der Hautalterung, die zwischen 0,01% und 20% wenigstens eines Wirkstoffs enthält, der aus dem Pulver der Knollen von Cyperus esculentus hervorgegangen ist.

12. Kosmetische und/oder dermopharmazeutische Zusammensetzung nach Anspruch 11, **dadurch gekennzeichnet, dass** diese in Gestalt einer Creme, einer Emulsion Öl-in-Wasser, einer Emulsion Wasser-in-Öl, mehrfacher Emulsionen, einer Lösung, einer Suspension oder eines Pulvers vorliegt.

13. Kosmetische und/oder dermopharmazeutische Zusammensetzung nach Anspruch 11 oder 12, **dadurch gekennzeichnet, dass** der Wirkstoff ein Wirkstoff nach einem der Ansprüche 5 bis 9 ist.

## Claims

1. Use of at least one active ingredient derived from powder of *Cyperus esculentus* tubers in or for the preparation of a cosmetic and/or dermopharmaceutical composition for topical administration for combatting skin ageing.

2. Use of at least one active ingredient derived from *Cyperus esculentus* according to claim 1, **characterised in that** said active ingredient is capable of acting on the synthesis of at least one specific fibrous molecule of the papillary dermis chosen from the functional collagens XII and/or XVI, oxytalan fibres and fibrillin-1 of the papillary dermis.

3. Use of at least one active ingredient derived from *Cyperus esculentus* according to any one of the preceding claims, **characterised in that** said active ingredient is capable of increasing the expression of the functional collagens XII and/or XVI and/or oxytalan fibres and/or fibrillin-1 of the papillary dermis.

4. Use of at least one active ingredient derived from *Cyperus esculentus* according to any one of the preceding claims, **characterised in that** said active ingredient is capable of increasing the expression of the matrix collagens I and/or VI.

5. An active ingredient suited for a use according to any one of the preceding claims, able to be obtained by a method comprising the following steps:
- solubilisation of powder of *Cyperus esculentus* tubers in water,
- successive enzymatic hydrolyses, by means of two carbohydrases,
- separation of the soluble and insoluble phases, and
- enzymatic inactivation by heat treatment.

6. An active ingredient according to claim 5, **characterised by**:
- a proportion of dry matter of between 10 and 250 g/l,
- a pH of between 3 and 7, and
- a total sugar content of between 8 and 240 g/l.

7. An active ingredient according to claim 5 or 6, **characterised by** a proportion of dry matter of between 38 and 52 g/l.

8. An active ingredient according to any one of claims 5 to 7, **characterised by** a pH of between 4.5 and 5.5.

9. The active principle according to any one of claims 5 to 8, **characterised by** a total sugar content of between 32 and 50 g/l.

10. A method of obtaining an active ingredient according to any one of claims 5 to 9, comprising the following steps:
- solubilisation of powder of *Cyperus esculentus* tubers in water,
- successive enzymatic hydrolyses, by means of two carbohydrases,
- separation of the soluble and insoluble phases,
- enzymatic inactivation by heat treatment, and
- filtration(s).

11. A cosmetic and/or dermopharmaceutical composition for topical administration intended to combat skin ageing, containing between 0.01% and 20% of at least one active ingredient derived from powder of *Cyperus esculentus* tubers.

12. A cosmetic and/or dermopharmaceutical composition according to claim 11, **characterised in that** it is in the form of cream, oil-in-water emulsion, water-in-oil emulsion, multiple emulsions, solution, suspension or powder.

13. A cosmetic and/or dermopharmaceutical composition according to claim 11 or 12, **characterised in that** the active ingredient is an active ingredient according to any one of claims 5 to 9.
